# EUROPEAN PATENT APPLICATION

(11) **EP 4 653 524 A1**
(43) Date of publication of application: **26.11.2025**
(21) Application number: 24752907.6
(22) Date of filing: 07.02.2024
(51) Int. Cl.: C12N 5/10, C12N 15/90, A61P 35/00, A61K 35/17

(54) **FUNCTION-ENHANCED CELL THERAPY**

(30) Priority: 10.02.2023 CN 202310096148
(71) Applicant: Nanjing Bioheng Biotech Co., Ltd, Nanjing, Jiangsu 210061 (CN)
(72) Inventor: ZHOU, Yali, Nanjing, Jiangsu 210061 (CN); TANG, Yingxiu, Nanjing, Jiangsu 210061 (CN); HOU, Jianqiang, Nanjing, Jiangsu 210061 (CN); ZHENG, Dong, Nanjing, Jiangsu 210061 (CN); REN, Jiangtao, Nanjing, Jiangsu 210061 (CN)
(74) Representative: Rimini, Rebecca
(86) International application number: PCT/CN2024/076741
(87) International publication number: WO 2024/165053

(57) **Abstract**

The present disclosure relates to an engineered cell that expresses an exogenous immunosuppressive molecule and expression of at least one endogenous gene thereof selected from the group consisting of: Fas, TNFR1, DR3, DR4, DR5, TGFBR1 and TGFBR2 is down-regulated. The present disclosure further relates to the engineered cell and a composition comprising the cell.

## Description

### CROSS-REFERENCE TO RELATED APPLICATION

The present disclosure claims the priority to the Chinese patent application with the application No. CN2023100961489 and entitled "FUNTION-ENHANCED CELL THERAPY", filed with China National Intellectual Patent Administration on February 10, 2023, which is incorporated in the present disclosure by reference in its entirety.

### TECHNICAL FIELD

The present disclosure pertains to the field of immunotherapy. Specifically, the present disclosure relates to an engineered cell that enhances efficacy and reduces the risk of rejection by down-regulating the expression of certain endogenous genes while expressing immunosuppressive molecules and a composition comprising the cell.

### BACKGROUND ART

As an emerging immunotherapy, adoptive cell therapy has developed very rapidly in recent years. Particularly for chimeric antigen receptor therapy, many products have been launched and have shown excellent therapeutic effects in clinical practice. However, the products currently on the market are all of autologous therapies, which have disadvantages such as long preparation times, high costs, and high preparation failure rates. In this regard, allogeneic cell therapies have been developed to improve patient accessibility. However, allogeneic cell therapies also face a series of challenges, for example, risk of immune rejection, poor cell persistence, etc. Therefore, there is still a need to improve the existing allogeneic cell therapies to reduce risk of immune rejection and enhance cell persistence.

### SUMMARY

Unless otherwise specified, all scientific and technical terms used herein have the same meaning as commonly understood by one of ordinary skill in the art to which the present disclosure pertains.

In a first aspect, the present disclosure provides an engineered cell comprising (i) the expression of at least one of the endogenous gene selected from the group consisting of: Fas, TNFR1, DR3, DR4, DR5, TGFBR1, TGFBR2 is down-regulated; and (ii) an exogenous immunosuppressive molecule is expressed, wherein the immunosuppressive molecule comprises one or more immunosuppressive protein-binding domains, a transmembrane domain and a co-stimulatory domain, and does not comprise a primary signaling domain, wherein the immunosuppressive protein-binding domain binds to an immunosuppressive protein selected from the group consisting of: PD1, NKG2A, FasL and CTLA4.

### Down-regulation of the expression of endogenous genes

The expression of at least one of the endogenous gene of the engineered cell provided in the present disclosure is down-regulated, wherein the endogenous gene is selected from Fas, TNFR1, DR3, DR4, DR5, TGFBR1 and TGFBR2.

Fas, TNFR1, DR3, DR4 and DR5 all pertain to the death receptor (DR) family, and share a Cys-rich extracellular domain and an intracellular death domain (DD). When these death receptors bind to their specific death ligands, they receive extracellular death signals, activate the intracellular apoptotic mechanism, and induce apoptosis. TGFBR1 and TGFBR2 are both receptors for TRFb1, and both contain serine/threonine kinase activity in the intracellular regions. They mediate Smad-dependent and -independent signaling pathways, and play an important regulatory role in processes such as cell proliferation, differentiation, wound healing, and tumorigenesis and metastasis. The inventors found that down-regulating the expression of these endogenous genes in cells is capable of significantly prolonging cell persistence and improving cell survival rate.

As used herein, gene expression being "down-regulated" refers to that the expression level of the product of the modified gene is significantly reduced or almost not expressed relative to the expression level of the unmodified wild-type gene. This means that for a gene whose expression is "down-regulated", the content of the expression product thereof (such as mRNA or protein) is reduced or the expression product lacks functionality. Therefore, down-regulation can be achieved in different dimensions such as gene level, transcription level, and translation level. Generally, gene expression is down-regulated by methods well known to those skilled in the art, including but not limited to, for example, knocking down or knocking out a gene of interest itself or the regulatory gene of the gene of interest by technologies such as meganucleases, zinc finger nucleases, TALEN, CRISPR/Cas systems, base editors, and lead editors, or reducing the expression level of the gene of interest itself or the regulatory gene of the gene of interest by technologies such as antisense oligonucleotides, RNAi, shRNA, transposons, siRNA, antigomer RNA, mutations, antibodies, or chemical inhibitors.

### Immunosuppressive molecules

The engineered cell provided in the present disclosure further expresses an exogenous immunosuppressive molecule, wherein the immunosuppressive molecule comprises one or more immunosuppressive protein-binding domains, a transmembrane domain and a co-stimulatory domain, and does not comprise a primary signaling domain, wherein the immunosuppressive protein-binding domain binds to an immunosuppressive protein selected from the group consisting of: PD1, NKG2A, FasL, CTLA4.

As used herein, the term "immunosuppressive molecule" refers to a molecule capable of binding to an immunosuppressive protein (e.g. PD1, NKG2A) and in turn to suppress immune rejection of exogenous cells by a subject, for example, reducing the killing function of immune cells (e.g., T cells and NK cells) in the subject or suppressing the excessive proliferation of immune cells.

In an embodiment, the immunosuppressive protein-binding domain is an antibody or ligand that specifically binds to an immunosuppressive protein. In an embodiment, the immunosuppressive protein-binding domain comprises an anti-PD1 antibody, an anti-NKG2A antibody, an anti-FasL antibody, an anti-CTLA4 antibody, an extracellular region of PDL1, an extracellular region of PDL2, an extracellular region of HLA-E, an extracellular region of Fas, an extracellular region of CD80, an extracellular region of CD86, or a combination thereof.

As used herein, the term "antibody" has the broadest meaning understood by those skilled in the art and includes monoclonal antibodies (comprising intact antibodies), polyclonal antibodies, multivalent antibodies, multispecific antibodies (e.g. bispecific antibodies), and antibody fragments or synthetic polypeptides carrying one or more CDR sequences capable of exhibiting the desired biological activity. The antibody of the present disclosure may be of any class (e.g. IgG, IgE, IgM, IgD, IgA, etc.) or subclass (e.g. IgG1, IgG2, IgG2a, IgG3, IgG4, IgA1, IgA2, etc.). As used herein, the term "antibody fragment" refers to one or more fragments of an antibody that retain the ability to specifically bind an antigen. It has been shown that the antigen binding function of an antibody can be performed by the fragment of a full-length antibody. Examples of antibody fragments of the present disclosure include, but are not limited to: Fab, Fab', F(ab')2, Fd fragment, Fd', Fv fragment, single-chain antibody (scFv), disulfide-linked Fv (sdFv), antibody heavy chain variable region (VH) or light chain variable region (VL), linear antibody, "diabody" with two antigen binding sites, single domain antibody (sdAb), nanobody, etc. Therefore, unless the context clearly dictates otherwise, the "antibody" of the present disclosure encompasses antibody fragments as defined above. Therefore, in a preferred embodiment, the antibody of the present disclosure is selected from the group consisting of IgG, Fab, Fab', F(ab')₂, Fd, Fd', Fv, scFv, sdFv, linear antibody, diabody, sdAb or nanobody, preferably scFv, sdAb or nanobody.

Typically, an intact antibody comprises two heavy chains and two light chains disulfide-linked together, each light chain being disulfide-linked to a respective heavy chain, to form a "Y" configuration. Each heavy chain comprises a heavy chain variable region (VH) and a heavy chain constant region, wherein the heavy chain variable region comprises three complementarity determining regions (CDRs): CDR1-H, CDR2-H and CDR3-H, and the heavy chain constant region comprises three constant domains: CH1, CH2 and CH3. Each light chain comprises a light chain variable region (VL) and a light chain constant region, wherein the light chain variable region comprises three CDRs: CDR1-L, CDR2-L and CDR3-L, and the light chain constant region comprises a constant domain CL. In the heavy/light chain variable regions, the CDRs are separated by more conserved framework regions (FRs). The heavy/light chain variable regions are responsible for the recognition and binding to the antigen, while the constant regions can mediate the binding of the antibody to host tissues or factors, including various cells of the immune system (e.g., effector cells) and the first component of the classical complement system.

The precise amino acid sequence boundaries for a given CDR or FR can be readily determined using a number of numbering schemes well known in the art, these schemes including: Kabat et al. (1991), "Sequences of Proteins of Immunological Interest," 5th Edition, Public Health Service, National Institutes of Health, Bethesda, MD ("Kabat" numbering scheme); Al-Lazikani et al., (1997) JMB 273, 927-948 ("Chothia" numbering scheme); MacCallum et al., J. Mol. Biol 262:732-745 (1996), "Antibody-antigen interactions: Contact analysis and binding sitetopography," J. Mol. Biol. 262, 732-745" ("Contact" numbering scheme); Lefranc MP et al., "IMGT unique numbering for immunoglobulin and T cell receptor variable domains and Ig superfamily V-like domains," Dev Comp Immunol, 2003 Jan; 27(1):55-77 ("IMGT" numbering scheme); Honegger A and Plückthun A, "Yet another numbering scheme for immunoglobulin variable domains: an automatic modeling and analysis tool," JMol Biol, 2001 Jun 8; 309(3):657-70 ("Aho" numbering scheme); and Martin et al., "Modeling antibody hypervariable loops: a combined algorithm," PNAS, 1989, 86(23):9268-9272 ("AbM" numbering scheme).

The boundaries of a given CDR or FR may vary depending on the scheme used for definition. For example, the Kabat scheme is based on structural alignment, while the Chothia scheme is based on structural information. Both the Kabat and Chothia numbering schemes are based on the sequence length of the most common antibody regions wherein insertions are provided by caret letters (e.g. "30a") and deletions occur in some antibodies. These two schemes place certain insertions and deletions (indels) at different positions, thereby resulting in different numbering. The Contact scheme is based on the analysis of complex crystal structures and is similar in many respects to the Chothia numbering scheme. The AbM scheme is a compromise between the Kabat and Chothia definitions and is based on the scheme used by the AbM antibody modeling software of Oxford Molecular.

Therefore, unless otherwise specified, a "CDR" of a given antibody or region thereof (e.g. variable region thereof) is understood to encompass the CDRs as defined by any of the above schemes or other known schemes. For example, where it is specified that a particular CDR (e.g. CDR3) comprises a given amino acid sequence, it is understood that such a CDR may also have the sequence of the corresponding CDR (e.g. CDR3) as defined by any of the above schemes or other known schemes. Likewise, unless otherwise specified, an FR for a given antibody or region thereof (e.g. variable region thereof) is understood to encompass the FRs as defined by any of the above schemes or other known schemes. Unless otherwise specified, the numbering scheme used herein to define the boundaries of CDRs and FRs adopts the Chothia scheme.

In an embodiment, the antibody of the present disclosure is a murine antibody, a chimeric antibody, a camelid antibody, a humanized antibody or a human antibody.

In an embodiment, the immunosuppressive protein-binding domain of the present disclosure is a PD1-targeting antibody. PD1 is a type I transmembrane protein composed of 288 amino acids, including an extracellular IgV domain, a transmembrane region, and an intracellular region, the intracellular region thereof containing an immunoreceptor tyrosine-based inhibitory motif (ITIM). PD-1 is expressed on the surface of activated T cells, B cells, and macrophages, and is widely involved in the negative regulation of the immune reaction of an organism. PD1 up-regulates E3-ubiquitin ligases CBL-b and c-CBL by binding to the ligands thereof (e.g. PDL1 or PDL2), triggering T cell receptor down-regulation and suppressing T cell activation and cytokine release. Studies have found that immune regulation targeting PD-1 is of great significance for anti-tumor, anti-infection, anti-autoimmune diseases, and graft survival in organ transplantation, etc.

Anti-PD1 antibodies known in the art can all be used in the present disclosure. In an embodiment, the PD1-targeting antibody comprises a light chain variable region and a heavy chain variable region, wherein the CDR1-L, CDR2-L and CDR3-L comprised in the light chain variable region are the same as the CDR1-L, CDR2-L and CDR3-L as comprised in SEQ ID NO: 51; wherein the CDR1-H, CDR2-H and CDR3-H comprised in the heavy chain variable region are the same as the CDR1-H, CDR2-H and CDR3-H as comprised in SEQ ID NO: 50. In an embodiment, CDR1-L comprised in the light chain variable region is as set forth in SEQ ID NO: 47, CDR2-L is as set forth in SEQ ID NO: 48, and CDR3-L is as set forth in SEQ ID NO: 49; CDR1-H comprised in the heavy chain variable region is as set forth in SEQ ID NO: 44, CDR2-H is as set forth in SEQ ID NO: 45, and CDR3-H is as set forth in SEQ ID NO: 46.

In an embodiment, the PD1-targeting antibody comprises a light chain variable region and a heavy chain variable region, wherein the light chain variable region has at least 90%, at least 91%, at least 92%, at least 93%, at least 94%, at least 95%, at least 96%, at least 97%, at least 98%, at least 99%, or 100% identity to SEQ ID NO: 51, or has modifications of one or more amino acids (e.g., at most 1, 2, 3, 4, 5, 6, 7, 8, 9 or 10 amino acids) compared to the amino acid sequence of SEQ ID NO: 51; wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 50, or has modifications of one or more amino acids (e.g., at most 1, 2, 3, 4, 5, 6, 7, 8, 9 or 10 amino acids) compared to the amino acid sequence as set forth in SEQ ID NO: 50. Preferably, the modifications are conservative modifications, for example, conservative substitutions, additions and deletions of amino acids. In a preferred embodiment, the anti-PD1 antibody of the present disclosure comprises a heavy chain variable region as set forth in SEQ ID NO: 50 and a light chain variable region as set forth in SEQ ID NO:51.

In an embodiment, the PD1-targeting antibody has at least 90%, at least 91%, at least 92%, at least 93%, at least 94%, at least 95%, at least 96%, at least 97%, at least 98%, at least 99%, or 100% identity to SEQ ID NO: 9 or 14, or has modifications of one or more amino acids (e.g., at most 1, 2, 3, 4, 5, 6, 7, 8, 9 or 10 amino acids) compared to the amino acid sequence as set forth in SEQ ID NO: 9 or 14. Preferably, the PD1-targeting antibody is as set forth in SEQ ID NO: 52.

In an embodiment, the immunosuppressive protein-binding domain comprised in the immunosuppressive molecule of the present disclosure is the extracellular region of a PD1 ligand, for example, the extracellular regions of PDL1 and PDL2. In this embodiment, the immunosuppressive molecule may further comprise the corresponding transmembrane region of PDL1 or PDL2, but generally does not comprise the corresponding intracellular region thereof at the same time. That is, the immunosuppressive molecule is not full-length PDL1 or PDL2. In this embodiment, the immunosuppressive molecule may further comprise a signal peptide of PDL1 or a signal peptide of PDL2.

In an embodiment, the extracellular region of PDL1 has at least 90%, at least 91%, at least 92%, at least 93%, at least 94%, at least 95%, at least 96%, at least 97%, at least 98%, at least 99%, or 100% identity to SEQ ID NO: 55; the extracellular region of PDL2 has at least 90%, at least 91%, at least 92%, at least 93%, at least 94%, at least 95%, at least 96%, at least 97%, at least 98%, at least 99%, or 100% identity to SEQ ID NO: 58. The transmembrane region of PDL1 has at least 90%, at least 91%, at least 92%, at least 93%, at least 94%, at least 95%, at least 96%, at least 97%, at least 98%, at least 99%, or 100% identity to SEQ ID NO: 54; the transmembrane region of PDL2 has at least 90%, at least 91%, at least 92%, at least 93%, at least 94%, at least 95%, at least 96%, at least 97%, at least 98%, at least 99%, or 100% identity to SEQ ID NO: 57. The PDL1 signal peptide has at least 90%, at least 91%, at least 92%, at least 93%, at least 94%, at least 95%, at least 96%, at least 97%, at least 98%, at least 99%, or 100% identity to SEQ ID NO: 53; the PDL2 signal peptide has at least 90%, at least 91%, at least 92%, at least 93%, at least 94%, at least 95%, at least 96%, at least 97%, at least 98%, at least 99%, or 100% identity to SEQ ID NO: 56.

In an embodiment, the immunosuppressive protein-binding domain comprised in the immunosuppressive molecule of the present disclosure is an NKG2A-targeting antibody. NKG2A is mainly expressed on NK cells and some T cells (CD8+T cells, Th2 cells and NKT cells). NKG2A forms a dimeric complex with CD94 and is recognized by its ligand HLA-E (a non-classical HLA class I molecule), recruiting SHP1 or SHP-2 through two ITIMs in its cytoplasmic tail, thereby inducing inhibitory signals and suppressing the cytotoxic activity of NK and the secretion of cytokines. Anti-NKG2A antibodies known in the art can all be used in the present disclosure, for example, Z270 (available from Immunotech, France), Z199 (available from Beckman Coulter, USA), 20D5 (available from BD Biosciences Pharmingen, USA), P25 (available from Morettaetal, Univ.Genova, Italy), etc.

In an embodiment, the NKG2A-targeting antibody comprises a light chain variable region and a heavy chain variable region, wherein the CDR1-L, CDR2-L and CDR3-L comprised in the light chain variable region are the same as the CDR1-L, CDR2-L and CDR3-L as comprised in SEQ ID NO: 40; wherein the CDR1-H, CDR2-H and CDR3-H comprised in the heavy chain variable region are the same as the CDR1-H, CDR2-H and CDR3-H as comprised in SEQ ID NO: 39. In an embodiment, CDR1-L comprised in the light chain variable region is as set forth in SEQ ID NO: 36, CDR2-L is as set forth in SEQ ID NO: 37, and CDR3-L is as set forth in SEQ ID NO: 38; CDR1-H comprised in the heavy chain variable region is as set forth in SEQ ID NO: 33, CDR2-H is as set forth in SEQ ID NO: 34, and CDR3-H is as set forth in SEQ ID NO: 35.

In an embodiment, the NKG2A-targeting antibody comprises a light chain variable region and a heavy chain variable region, wherein the light chain variable region has at least 90%, at least 91%, at least 92%, at least 93%, at least 94%, at least 95%, at least 96%, at least 97%, at least 98%, at least 99%, or 100% identity to SEQ ID NO: 40, or has modifications of one or more amino acids (e.g., at most 1, 2, 3, 4, 5, 6, 7, 8, 9 or 10 amino acids) compared to the amino acid sequence of SEQ ID NO: 40; wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 39, or has modifications of one or more amino acids (e.g., at most 1, 2, 3, 4, 5, 6, 7, 8, 9 or 10 amino acids) compared to the amino acid sequence as set forth in SEQ ID NO: 39. Preferably, the modifications are conservative modifications, for example, conservative substitutions, additions and deletions of amino acids. In a preferred embodiment, the anti-NKG2A antibody of the present disclosure comprises a heavy chain variable region as set forth in SEQ ID NO: 39 and a light chain variable region as set forth in SEQ ID NO:40.

In an embodiment, the NKG2A-targeting antibody has at least 90%, at least 91%, at least 92%, at least 93%, at least 94%, at least 95%, at least 96%, at least 97%, at least 98%, at least 99%, or 100% identity to SEQ ID NO: 41, or has modifications of one or more amino acids (e.g., at most 1, 2, 3, 4, 5, 6, 7, 8, 9 or 10 amino acids) compared to the amino acid sequence as set forth in SEQ ID NO: 58. Preferably, the NKG2A-targeting antibody is as set forth in SEQ ID NO: 41.

In an embodiment, the immunosuppressive protein-binding domain of the present disclosure comprises the extracellular region of an NKG2A ligand, for example, the extracellular region of HLA-E. In an embodiment, the extracellular region of HLA-E has at least 90%, at least 91%, at least 92%, at least 93%, at least 94%, at least 95%, at least 96%, at least 97%, at least 98%, at least 99%, or 100% identity to SEQ ID NO: 42 or 43. In this embodiment, the immunosuppressive molecule may further comprise the corresponding transmembrane region of HLA-E, but generally does not comprise the corresponding intracellular region thereof at the same time. That is, the immunosuppressive molecule is not full-length HLA-E.

In an embodiment, the immunosuppressive protein-binding domain of the present disclosure is a FasL-targeting antibody. FasL is a type II cell membrane surface glycoprotein, pertaining to a member of the tumor necrosis factor receptor superfamily (TNFRSF). FasL is distributed on the surface of activated T lymphocytes, NK cells, mononuclear macrophages, etc., and its ligand is Fas. It has been found that the Fas/FasL system is one of the important pathways of cell apoptosis. Specifically, the binding of FasL to Fas will form a death-inducing complex, which in turn activates the caspase signaling pathway, and ultimately leads to cell apoptosis through the phosphorylation of tyrosine and serine in the cells.

Anti-FasL antibodies known in the art can all be used in the present disclosure. In an embodiment, the FasL-targeting antibody comprises a light chain variable region and a heavy chain variable region, wherein CDR1-L, CDR2-L and CDR3-L comprised in the light chain variable region are the same as the CDR1-L, CDR2-L and CDR3-L comprised in SEQ ID NO: 66; wherein CDR1-H, CDR2-H and CDR3-H comprised in the heavy chain variable region are the same as CDR1-H, CDR2-H and CDR3-H comprised in SEQ ID NO: 65. In an embodiment, CDR1-L comprised in the light chain variable region is as set forth in SEQ ID NO: 62, CDR2-L is as set forth in SEQ ID NO: 63, and CDR3-L is as set forth in SEQ ID NO: 64; CDR1-H comprised in the heavy chain variable region is as set forth in SEQ ID NO: 59, CDR2-H is as set forth in SEQ ID NO: 60, and CDR3-H is as set forth in SEQ ID NO: 61.

In an embodiment, the FasL-targeting antibody comprises a light chain variable region and a heavy chain variable region, wherein the light chain variable region has at least 90%, at least 91%, at least 92%, at least 93%, at least 94%, at least 95%, at least 96%, at least 97%, at least 98%, at least 99%, or 100% identity to SEQ ID NO: 66, or has modifications of one or more amino acids (e.g., at most 1, 2, 3, 4, 5, 6, 7, 8, 9 or 10 amino acids) compared to the amino acid sequence of SEQ ID NO: 66; wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 65, or has modifications of one or more amino acids (e.g., at most 1, 2, 3, 4, 5, 6, 7, 8, 9 or 10 amino acids) compared to the amino acid sequence as set forth in SEQ ID NO: 65. Preferably, the modifications are conservative modifications, for example, conservative substitutions, additions and deletions of amino acids. In a preferred embodiment, the anti-FasL antibody of the present disclosure comprises a heavy chain variable region as set forth in SEQ ID NO: 65 and a light chain variable region as set forth in SEQ ID NO:66.

In an embodiment, the FasL-targeting antibody has at least 90%, at least 91%, at least 92%, at least 93%, at least 94%, at least 95%, at least 96%, at least 97%, at least 98%, at least 99%, or 100% identity to SEQ ID NO: 67, or has modifications of one or more amino acids (e.g., at most 1, 2, 3, 4, 5, 6, 7, 8, 9 or 10 amino acids) compared to the amino acid sequence as set forth in SEQ ID NO: 67. Preferably, the FasL-targeting antibody is as set forth in SEQ ID NO: 67.

In an embodiment, the immunosuppressive protein-binding domain of the present disclosure comprises the extracellular region of a FasL ligand, for example, the extracellular region of Fas. In an embodiment, the extracellular region of Fas has at least 90%, at least 91%, at least 92%, at least 93%, at least 94%, at least 95%, at least 96%, at least 97%, at least 98%, at least 99%, or 100% identity to SEQ ID NO: 68. In this embodiment, the immunosuppressive molecule may further comprise the corresponding transmembrane region of Fas, but generally does not comprise the corresponding intracellular region thereof at the same time. That is, the immunosuppressive molecule is not full-length Fas. In an embodiment, the transmembrane region of Fas has at least 90%, at least 91%, at least 92%, at least 93%, at least 94%, at least 95%, at least 96%, at least 97%, at least 98%, at least 99%, or 100% identity to SEQ ID NO: 69.

In an embodiment, the immunosuppressive protein-binding domain of the present disclosure is a CTLA4-targeting antibody. CTLA4, also known as CD152, is mainly expressed on the surface of activated T cells. It has a high degree of homology with CD28, a costimulatory molecule receptor on the surface of T cells, but its affinity for the same ligands CD80 and CD86 is significantly higher than that of CD28. In contrast to the activation functions of CD28, such as promoting cytokine production, cell survival, cell differentiation, etc., CTLA4 transmits inhibitory signals to T cells.

Anti-CTLA4 antibodies known in the art can all be used in the present disclosure. In an embodiment, the CTLA4-targeting antibody comprises a light chain variable region and a heavy chain variable region, wherein the CDR1-L, CDR2-L and CDR3-L comprised in the light chain variable region are the same as the CDR1-L, CDR2-L and CDR3-L as comprised in SEQ ID NO: 77; wherein the CDR1-H, CDR2-H and CDR3-H comprised in the heavy chain variable region are the same as the CDR1-H, CDR2-H and CDR3-H as comprised in SEQ ID NO: 76. In an embodiment, CDR1-L comprised in the light chain variable region is as set forth in SEQ ID NO: 73, CDR2-L is as set forth in SEQ ID NO: 74, and CDR3-L is as set forth in SEQ ID NO: 75; CDR1-H comprised in the heavy chain variable region is as set forth in SEQ ID NO: 70, CDR2-H is as set forth in SEQ ID NO: 71, and CDR3-H is as set forth in SEQ ID NO: 72.

In an embodiment, the CTLA4-targeting antibody comprises a light chain variable region and a heavy chain variable region, wherein the light chain variable region has at least 90%, at least 91%, at least 92%, at least 93%, at least 94%, at least 95%, at least 96%, at least 97%, at least 98%, at least 99%, or 100% identity to SEQ ID NO: 77, or has modifications of one or more amino acids (e.g., at most 1, 2, 3, 4, 5, 6, 7, 8, 9 or 10 amino acids) compared to the amino acid sequence of SEQ ID NO: 77; wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 76, or has modifications of one or more amino acids (e.g., at most 1, 2, 3, 4, 5, 6, 7, 8, 9 or 10 amino acids) compared to the amino acid sequence as set forth in SEQ ID NO: 76. Preferably, the modifications are conservative modifications, for example, conservative substitutions, additions and deletions of amino acids. In a preferred embodiment, the anti-CTLA4 antibody of the present disclosure comprises a heavy chain variable region as set forth in SEQ ID NO: 76 and a light chain variable region as set forth in SEQ ID NO:77.

In an embodiment, the CTLA4-targeting antibody has at least 90%, at least 91%, at least 92%, at least 93%, at least 94%, at least 95%, at least 96%, at least 97%, at least 98%, at least 99%, or 100% identity to SEQ ID NO: 78, or has modifications of one or more amino acids (e.g., at most 1, 2, 3, 4, 5, 6, 7, 8, 9 or 10 amino acids) compared to the amino acid sequence as set forth in SEQ ID NO: 78. Preferably, the CTLA4-targeting antibody is as set forth in SEQ ID NO: 78.

In an embodiment, the immunosuppressive protein-binding domain of the present disclosure comprises the extracellular region of a CTLA4 ligand, for example, the extracellular region of CD80 or CD86. In an embodiment, the extracellular region of CD80 has at least 90%, at least 91%, at least 92%, at least 93%, at least 94%, at least 95%, at least 96%, at least 97%, at least 98%, at least 99%, or 100% identity to SEQ ID NO: 79; the extracellular region of CD86 has at least 90%, at least 91%, at least 92%, at least 93%, at least 94%, at least 95%, at least 96%, at least 97%, at least 98%, at least 99%, or 100% identity to SEQ ID NO: 80. In this embodiment, the immunosuppressive molecule may further comprise the corresponding transmembrane region of CD80 or CD86, but generally does not comprise the corresponding intracellular region thereof at the same time. That is, the immunosuppressive molecule is not full-length CD80 or CD86. In an embodiment, the transmembrane region of CD80 has at least 90%, at least 91%, at least 92%, at least 93%, at least 94%, at least 95%, at least 96%, at least 97%, at least 98%, at least 99%, or 100% identity to SEQ ID NO: 81; the transmembrane region of CD86 has at least 90%, at least 91%, at least 92%, at least 93%, at least 94%, at least 95%, at least 96%, at least 97%, at least 98%, at least 99%, or 100% identity to SEQ ID NO: 82.

In an embodiment, the engineered cell expresses an exogenous immunosuppressive molecule comprising an anti-PD1 antibody, an anti-NKG2A antibody, an anti-FasL antibody, an anti-CTLA4 antibody, an extracellular region of Fas, an extracellular region of CD80, an extracellular region of CD86, an extracellular region of PDL1, an extracellular region of PDL2, an extracellular region of HLA-E, or a combination thereof, for example, the combination of an anti-NKG2A antibody (or an extracellular region of HLA-E) and an anti-PD1 antibody (or an extracellular region of PDL1, an extracellular region of PDL2), an anti-NKG2A antibody (or an extracellular region of HLA-E) and an anti-FasL antibody (or an extracellular region of Fas), an anti-NKG2A antibody (or an extracellular region of HLA-E) and an anti-CTLA4 antibody (or an extracellular region of CD80, an extracellular region of CD86), an anti-PD1 antibody (or an extracellular region of PDL1, an extracellular region of PDL2) and an anti-FasL antibody (or an extracellular region of Fas), an anti-PD1 antibody (or an extracellular region of PDL1, an extracellular region of PDL2) and an anti-CTLA4 antibody (or an extracellular region of CD80, an extracellular region of CD86), or an anti-FasL antibody (or an extracellular region of Fas) and an anti-CTLA4 antibody (or an extracellular region of CD80, an extracellular region of CD86) (the two binding domains in the combination may be linked by a linker).

In an embodiment, the engineered cell expresses two exogenous immunosuppressive molecules, each of which comprises a binding domain that binds a different immunosuppressive protein. For example, the two exogenous immunosuppressive molecules bind NKG2A and PD1, NKG2A and CTLA4, NKG2A and FasL, PD1 and CTLA4, PD1 and FasL, or CTLA4 and FasL, respectively. For example, In an embodiment, the engineered cell expresses two exogenous immunosuppressive molecules, which comprise an anti-NKG2A antibody (or an extracellular region of HLA-E) and an anti-PD1 antibody (or an extracellular region of PDL1, an extracellular region of PDL2), an anti-NKG2A antibody (or an extracellular region of HLA-E) and an anti-FasL antibody (or an extracellular region of Fas), an anti-NKG2A antibody (or an extracellular region of HLA-E) and an anti-CTLA4 antibody (or an extracellular region of CD80, an extracellular region of CD86), an anti-PD1 antibody (or an extracellular region of PDL1, an extracellular region of PDL2) and an anti-FasL antibody (or an extracellular region of Fas), an anti-PD1 antibody (or an extracellular region of PDL1, an extracellular region of PDL2) and an anti-CTLA4 antibody (or an extracellular region of CD80, an extracellular region of CD86), or an anti-FasL antibody (or an extracellular region of Fas) and an anti-CTLA4 antibody (or an extracellular region of CD80, an extracellular region of CD86), respectively. In this embodiment, the two exogenous immunosuppressive molecules can be linked by a 2A peptide (e.g., F2A, T2A, etc.). As used herein, "A (or B) and C (or D, E)" encompasses various combinations of A and C, A and D, A and E, B and C, B and D, and B and E, etc.

As used herein, the term "conservative modification" refers to an amino acid modification that does not significantly affect or alter the binding characteristics of an antibody or antibody fragment comprising the amino acid sequence. These conservative modifications include conservative substitutions, additions and deletions of amino acids. Modifications can be introduced into the chimeric antigen receptor of the present disclosure by standard techniques known in the art, e.g., site-directed mutagenesis and PCR-mediated mutagenesis. A conservative amino acid substitution is one in which an amino acid residue is replaced by an amino acid residue with a similar side chain. Families of amino acid residues with similar side chains have been defined in the art and include those with basic side chain (e.g., lysine, arginine, histidine), acidic side chain (e.g., aspartic acid, glutamic acid), uncharged polar side chain (e.g., glycine, asparagine, glutamine, serine, threonine, tyrosine, cysteine), non-polar side chain (e.g., alanine, valine, leucine, isoleucine, proline, phenylalanine, methionine, tryptophan), beta-branched side chain (e.g., threonine, valine, isoleucine) and aromatic side chain (e.g., tyrosine, phenylalanine, tryptophan, histidine). Conservative modifications can be selected, for example, on the basis of similarity in polarity, charge, solubility, hydrophobicity, hydrophilicity, and/or the amphipathic nature of the residues involved.

As used herein, the term sequence "identity" means the degree to which two (nucleotide or amino acid) sequences in alignment have the same residue at the same position, and is usually expressed as a percentage. Preferably, identity is determined over the entire length of the sequences being compared. Therefore, two copies of the exact same sequence have 100% identity. Those skilled in the art know that several algorithms can be used to determine sequence identity, for example, Blast (Altschul et al. (1997) Nucleic Acids Res. 25:3389-3402), Blast2 (Altschul et al. (1990) J. Mol. Biol. 215: 403-410), Smith-Waterman (Smith et al. (1981) J. Mol. Biol. 147:195-197) and Clustal W.

As used herein, the term "transmembrane domain" refers to a polypeptide structure that enables the expression of an immunosuppressive molecule on the cell surface, and anchors the immunosuppressive protein-binding domain to the cell membrane. The transmembrane domain may be natural or synthetic, and also may be derived from any membrane-bound protein or transmembrane protein. The transmembrane domain is capable of signaling when a target binding domain binds to a target. The transmembrane domain particularly suitable for the present disclosure can be derived from, for example, a TCR α chain, a TCR β chain, a TCR γ chain, a TCR δ chain, a CD3 ζ subunit, a CD3 ε subunit, a CD3 γ subunit, a CD3 δ subunit, CD28, CD45, CD4, CD5, CD8a, CD9, CD16, CD22, CD33, CD37, CD47, CD64, CD80, CD86, CD94, CD134, CD137, CD154, KIRDS2, OX40, CD2, CD27, CD18, ICOS, 4-1BB, GITR, CD40, BAFFR, HVEM, SLAMF7, NKp80, CD160, BCMA, IL-2R β, IL-2R γ, IL-7R a, ITGA1, VLA1, CD49a, ITGA4, IA4, CD49D, ITGA6, VLA-6, CD49f, ITGAD, CD1 1d, ITGAE, CD103, ITGAL, CD1 1a, LFA-1, ITGAM, CD1 1b, ITGAX, CD1 1c, ITGB1, CD29, ITGB2, CD18, LFA-1, ITGB7, TNFR2, DNAM1, SLAMF4, CD84, CD96, CEACAM1, CRT AM, Ly9, CD160, PSGL1, CDIOO, SLAMF6, SLAMF1, SLAMF8, CD162, LTBR, PAG/Cbp, NKp44, NKp30, NKp46, NKG2D or NKG2C. In some embodiments, the transmembrane domain is derived from the following molecules: CD8a, CD4, CD28, 4-1BB, CD47, CD80, CD86, CD152 and PD1. Alternatively, the transmembrane domain may be synthetic and may mainly comprise hydrophobic residues, e.g., leucine and valine. Preferably, the transmembrane domain is derived from CD28, which has at least 70%, preferably at least 80%, more preferably at least 90%, 95%, 97%, or 99%, or 100% sequence identity to the amino acid sequence as set forth in SEQ ID NO: 14. Preferably, the transmembrane domain is derived from CD8a, which has at least 70%, preferably at least 80%, more preferably at least 90%, 95%, 97%, or 99%, or 100% sequence identity to the amino acid sequence as set forth in SEQ ID NO: 15 or 16.

In an embodiment, the immunosuppressive molecule further comprises a hinge region located between the immunosuppressive protein-binding domain and the transmembrane domain. As used herein, the term "hinge region" generally refers to any oligopeptide or polypeptide that functions to link a transmembrane domain to an antibody. Specifically, the hinge region serves to provide greater flexibility and accessibility to the antibody. The hinge region may comprise up to 300 amino acids, preferably 10 to 100 amino acids and most preferably 25 to 50 amino acids. The hinge region may be completely or partially derived from a natural molecule, e.g., completely or partially from the extracellular region of CD8, CD4 or CD28, or completely or partially from an antibody constant region. Alternatively, the hinge region may be a synthetic sequence corresponding to a naturally existing hinge sequence, or may be a completely synthetic hinge sequence. In a preferred embodiment, the hinge region comprises a hinge region moiety of CD8α, CD28, a FeγRIIIα receptor, IgG4 or IgG1, more preferably a hinge of CD8α, CD28 or IgG4. In an embodiment, the hinge region is from CD28, which has at least 70%, preferably at least 80%, more preferably at least 90%, 95%, 97%, or 99%, or 100% sequence identity to the amino acid sequence as set forth in SEQ ID NO: 26. In an embodiment, the hinge region is from CD8a, which has at least 70%, preferably at least 80%, more preferably at least 90%, 95%, 97%, or 99%, or 100% sequence identity to the amino acid sequence as set forth in SEQ ID NO: 27 or 28. In an embodiment, the hinge region is from IgG4, which has at least 70%, preferably at least 80%, more preferably at least 90%, 95%, 97%, or 99%, or 100% sequence identity to the amino acid sequence as set forth in SEQ ID NO: 29.

As used herein, a "co-stimulatory domain" refers to at least a moiety of a protein that mediates intracellular signal transduction to induce an immune reaction such as an effector function, which is an intracellular functional signaling domain from a co-stimulatory molecule, comprising the entire intracellular region of the co-stimulatory molecule, or a functional fragment thereof. A "co-stimulatory molecule" refers to a cognate binding partner that specifically binds to a co-stimulatory ligand, thereby mediating a co-stimulatory response (e.g., proliferation and survival). The co-stimulatory signaling domain of any co-stimulatory molecule is suitable for the immunosuppressive molecule as described herein. The co-stimulatory molecule includes, but is not limited to, MHC class 1 molecules, BTLA, and Toll ligand receptors. Non-limiting examples of the co-stimulatory domain of the present disclosure include, but are not limited to, a intracellular region derived from the following protein: LTB, CD94, TLR1, TLR2, TLR3, TLR4, TLR5, TLR6, TLR7, TLR8, TLR9, TLR10, CARD11, CD2, CD7, CD8, CD18, CD27, CD28, CD30, CD40, CD54, CD83, CD134, 4-1BB, CD270, CD272, B7-H3, ICOS, CD357, DAP10, DAP12, LAT, NKG2C, SLP76, PD-1, LIGHT, TRIM, ZAP70 and a combination thereof. Preferably, the co-stimulatory domain of the CAR of the present disclosure is 4-1BB, CD28 or 4-1BB+CD28. In an embodiment, the co-stimulatory domain is from 4-1BB, which has at least 70%, preferably at least 80%, more preferably at least 90%, 95%, 97%, or 99%, or 100% sequence identity to the amino acid sequence as set forth in SEQ ID NO: 18 or 19. In an embodiment, the co-stimulatory domain is from CD28, which has at least 70%, preferably at least 80%, more preferably at least 90%, 95%, 97%, or 99%, or 100% sequence identity to the amino acid sequence as set forth in SEQ ID NO: 17.

The immunosuppressive molecule of the present disclosure does not comprise a primary signaling domain. As used herein, the term "primary signaling domain" refers to a protein structure that acts together to initiate primary signaling upon antigen-receptor binding, which is generally an intracellular sequence of a T cell receptor and a co-receptor. The primary signaling domain generally comprises one or more Immunoreceptor Tyrosine-based Activation Motifs (ITAM). Non-limiting examples of the primary signaling domain of the present disclosure include, but are not limited to, those derived from FcRγ, FcRβ, CD3γ, CD3δ, CD3ε, CD3ζ, CD5, CD22, CD79a, CD79b, NFAM1, STAM1, STAM2, and CD66d. In an embodiment, the immunosuppressive molecule of the present disclosure does not comprise a CD3ζ intracellular region. For example, the CD3ζ intracellular region has at least 70%, preferably at least 80%, more preferably at least 90%, 95%, 97% or 99%, or 100% sequence identity to the amino acid sequence as set forth in SEQ ID NO: 21, 22 or 20.

In an embodiment, the immunosuppressive molecule of the present disclosure further comprises a signal peptide such that when it is expressed in a cell, e.g. a T cell, the nascent protein is directed to the endoplasmic reticulum and subsequently to the cell surface. The core of the signal peptide may contain a long hydrophobic amino acid segment, which has a tendency to form a single α-helix. At the end of the signal peptide, there is usually an amino acid segment capable of being recognized and cleaved by signal peptidase. The signal peptidase may cleave during or after translocation, so as to generate free signal peptide and mature protein. Then, the free signal peptide is digested by a specific protease. Signal peptides that may be used in the present disclosure are well known to those skilled in the art, for example, signal peptides derived from B2M, CD8α, IgG1, GM-CSFRα, etc. In an embodiment, the signal peptide that can be used in the present disclosure is from B2M, which has at least 70%, preferably at least 80%, more preferably at least 90%, 95%, 97%, or 99%, or 100% sequence identity to the amino acid sequence as set forth in SEQ ID NO: 23. In an embodiment, the signal peptide that can be used in the present disclosure is from CD8a, which has at least 70%, preferably at least 80%, more preferably at least 90%, 95%, 97%, or 99%, or 100% sequence identity to the amino acid sequence as set forth in SEQ ID NO: 24 or 25.

### Functional exogenous receptors

In an embodiment, the engineered cell of the present disclosure can further express a functional exogenous receptor. Preferably, the functional exogenous receptor is selected from a recombinant T cell receptor, a chimeric antigen receptor, a T cell fusion protein or a T cell antigen coupler, more preferably a chimeric antigen receptor.

As used herein, the term "T cell fusion protein" or "TFP" refers to a recombinant polypeptide derived from each component of a TCR, usually composed of a TCR subunit and an antibody linked thereto, and expressed on the cell surface. Wherein, the TCR subunit includes at least part of the TCR extracellular domain, the transmembrane domain, and the TCR intracellular signal domain.

As used herein, the term "T cell antigen coupler" or "TAC" includes three functional domains: 1 a tumor targeting domain, including a single-chain antibody, a designed ankyrin repeat protein (DARPin) or other targeting groups; 2 an extracellular region domain, a CD3-binding single-chain antibody, so that TAC receptors and TCR receptors are close to each other; 3 a transmembrane region and the intracellular region of a CD4 co-receptor, wherein the intracellular region is linked to protein kinase LCK, to catalyze the phosphorylation of the immunoreceptor tyrosine activation motif (ITAM) of the TCR complex as an initial step in T cell activation.

As used herein, the term "T cell receptor" or "TCR" is a characteristic marker on the surface of T cells that binds to CD3 with a non-covalent bond to form a complex. Antigen presenting cells present antigenic peptides to T cells through major histocompatibility complex molecules (MHC) and bind to TCR complexes to induce a series of intracellular signaling. TCR is composed of six peptide chains that form heterodimers, which are generally divided into αβ type and γδ type. Each peptide chain includes a constant region and a variable region, wherein the variable region is responsible for binding specific antigen and MHC molecules. The term "recombinant TCR receptor" refers to an artificially constructed T cell receptor that further comprises an antigen (e.g., a tumor antigen) binding domain.

As used herein, the term "chimeric antigen receptor" or "CAR" refers to an artificially constructed hybrid polypeptide that generally includes an antigen (for example, a tumor antigen) binding domain (for example, an antibody or a ligand of antigen), a transmembrane domain, an optional co-stimulatory domain, and a primary signaling domain, and each domain is linked by a linker. CARs are able to reposition the specificity and reactivity of T cells and other immune cells to a selected target in a non-MHC-restricted manner. In an embodiment, the functional exogenous receptor of the present disclosure is a chimeric antigen receptor, which comprises a tumor antigen binding domain, a transmembrane domain, one or more co-stimulatory domains, and a primary signaling domain. In an embodiment, the chimeric antigen receptor further comprises one or more of the following structures: a signal peptide, a hinge region, a suicide gene, a switch structure, etc.

In an embodiment, the functional exogenous receptor comprises an extracellular domain that specifically recognizes an antigen (e.g., a tumor antigen). In an embodiment, the extracellular domain comprises an antibody that specifically binds an antigen or a ligand of the antigen. In an embodiment, the antigen is selected from the group consisting of: ALK, ADRB3, AKAP-4, APRIL, ASGPR1, BCMA, B7H3, B7H4, B7H6, ber-abl, BORIS, BST2, BAFF-R, BTLA, CD2, CD3, CD4, CD5, CD7, CD8, CD19, CD20, CD22, CD24, CD25, CD28, CD30, CD33, CD38, CD40, CD44, CD44v6, CD44v7/8, CD47, CD52, CD56, CD57, CD58, CD70, CD72, CD79a, CD79b, CD80, CD81, CD86, CD97, CD123, CD133, CD137, CD 138, CD151, CD171, CD179a, CD300LF, CLEC12A, CDH16, CSPG4, CS1, CLL-1, Claudin 6, Claudin18.1, Claudin 18.2, CEA, CEACAM6, c-Met, CAIX, CXORF61, CA125, CYP1B1, CS1, ELF2M, EGFR, EPCAM, EGFRvIII, EphA2, ERG/TMPRSS2ETS fusion gene, ETV6-AML, EMR2, EGP2, EGP40, FAP, FAR, FBP, FLT3, FOSL1, FCRL5, FCAR, Flt3, Flt4, Frizzled, GD2, GD3, gp100, gp130, GM3, GPC2, GPC3, GPRC5D, GPR20, GloboH, GHRHR, GHR, GITR, Her2, HER3, HER-4, HMWMAA, HAVCR1, HPV E6,E7, HVEM, HIV-1Gag, HLA-A1, HLA-A2, IL6R, IL-11Ra, IL-13Ra, IGF-I receptor, LTPR, LIFRP, LRP5, IGLL1, IGF1R, KIT, Kappa Light Chain, KDR, LewisY, LMP2, LY6K, LAGE-1a, legumain, LCK, LAIR1, LILRA2, LY75, MSLN, MUC1, MUC16, MAGE-A1, MAGE3, MAD-CT-1, MelanA/MART1, ML-IAP, MYCN, mut hsp70-2, NCAM, NY-BR-1, NY-ESO-1, NA17, Notch-1-4, nAchR, NKG2D, NKG2D ligand, OY-TES1, OR51E2, OX40, PRSS21, PSCA, PD1, PD-L1, PD-L2, PSMA, Prostase, PAP, PDGFR-β, PCTA-1/galectin 8, p53, p53 mutant, prostein, PLAC1, PANX3, PAX3, PAX5, PTCH1, RANK, RAGE-1, ROR1, Ras mutant, RhoC, RU1, RU2, Robol, SSEA-4, SSX2, SART3, Sp17, TSHR, Tn Ag, TGS5, TEM1/CD248, TEM7R, TARP, TCRα, TCRβ, TGFBR1, TGFBR2, TNFRSF4, TWEAK-R, TLR7, TLR9, TAG72, TROP-2, Tie 2, TRP-2, TNFR1, TNFR2, TEM1, UPK2VEGFR, WT1, XAGE1, 5T4, 8H9, αvβ6 integrin, CA9, folate receptor α, ephrin B2, tyrosinase, fucosyl GM1, o-acetyl-GD2, folate receptor β, polysialic acid, sperm protein 17, survivin and telomerase, sarcoma translocation breakpoint, human telomerase reverse transcriptase/hTERT, androgen receptor, intestinal carboxylesterase, cyclin B1, fibronectin, tenascin, carcinoembryonic variant of tumor necrosis region, or any combination thereof. Preferably, the antigen is selected from CD7, CD19, CD20, CD22, CD30, CD33, CD38, CD123, CD138, CD171, MUC1, MSLN, AFP, folate receptor α, CEA, PSCA, PSMA, Her2, EGFR, IL-13Ra, GD2, NKG2D, Claudin18.2, ROR1, EGFRvIII, CS1, BCMA and GPRC5D, more preferably selected from ROR1, CD19, Claudin18.2, MSLN, GPRC5D, CD7 and BCMA.

In an embodiment, the functional exogenous receptor comprises an extracellular domain that specifically recognizes CD19, for example, a CD19-targeting antibody. In an embodiment, the CD19-targeting antibody comprises a light chain variable region and a heavy chain variable region, wherein CDR1-L comprised in the light chain variable region is as set forth in SEQ ID NO: 8, CDR2-L is as set forth in SEQ ID NO: 9, and CDR3-L is as set forth in SEQ ID NO: 10; CDR1-H comprised in the heavy chain variable region is as set forth in SEQ ID NO: 5, CDR2-H is set forth in SEQ ID NO: 6, and CDR3-H is as set forth in SEQ ID NO: 7.

In an embodiment, the CD19-targeting antibody comprises a light chain variable region and a heavy chain variable region, wherein the light chain variable region has at least 90%, at least 91%, at least 92%, at least 93%, at least 94%, at least 95%, at least 96%, at least 97%, at least 98%, at least 99%, or 100% identity to SEQ ID NO: 12, or has modifications of one or more amino acids (e.g., at most 1, 2, 3, 4, 5, 6, 7, 8, 9 or 10 amino acids) compared to the amino acid sequence of SEQ ID NO: 12; wherein the heavy chain variable region has at least 90%, at least 91%, at least 92%, at least 93%, at least 94%, at least 95%, at least 96%, at least 97%, at least 98%, at least 99%, or 100% identity to SEQ ID NO: 11, or has modifications of one or more amino acids (e.g., at most 1, 2, 3, 4, 5, 6, 7, 8, 9 or 10 amino acids) compared to the amino acid sequence as set forth in SEQ ID NO: 11. Preferably, the modifications are conservative modifications, for example, conservative substitutions, additions and deletions of amino acids. In a preferred embodiment, the antibody of the present disclosure comprises a heavy chain variable region as set forth in SEQ ID NO: 11 and a light chain variable region as set forth in SEQ ID NO: 12.

In an embodiment, the CD19-targeting antibody has at least 90%, at least 91%, at least 92%, at least 93%, at least 94%, at least 95%, at least 96%, at least 97%, at least 98%, at least 99%, or 100% identity to SEQ ID NO: 13, or has modifications of one or more amino acids (e.g., at most 1, 2, 3, 4, 5, 6, 7, 8, 9 or 10 amino acids) compared to the amino acid sequence as set forth in SEQ ID NO: 13. Preferably, the CD19-targeting antibody is as set forth in SEQ ID NO: 13.

In an embodiment, the functional exogenous receptor comprises an extracellular domain that specifically recognizes Claudin18.2, for example, a Claudin18.2-targeting antibody. In an embodiment, the Claudin18.2-targeting antibody is a single domain antibody. Preferably, the single domain antibody comprises CDR1 as set forth in SEQ ID NO: 1, CDR2 as set forth in SEQ ID NO: 2, and CDR3 as set forth in SEQ ID NO: 3.

In an embodiment, the Claudin18.2-targeting antibody has at least 90%, at least 91%, at least 92%, at least 93%, at least 94%, at least 95%, at least 96%, at least 97%, at least 98%, at least 99%, or 100% identity to SEQ ID NO: 4, or has modifications of one or more amino acids (e.g., at most 1, 2, 3, 4, 5, 6, 7, 8, 9 or 10 amino acids) compared to the amino acid sequence as set forth in SEQ ID NO: 4. Preferably, the Claudin18.2-targeting antibody is as set forth in SEQ ID NO: 4.

In an embodiment, the functional exogenous receptor of the present disclosure is a chimeric antigen receptor, which comprises an antigen binding domain, a transmembrane domain, one or more co-stimulatory domains, and a primary signaling domain. In an embodiment, the functional exogenous receptor of the present disclosure is a chimeric antigen receptor, the chimeric antigen receptor targeting CD19, CD7, CD19, CD20, CD22, CD30, CD33, CD38, CD123, CD138, CD171, MUC1, MSLN, AFP, folate receptor α, CEA, PSCA, PSMA, Her2, EGFR, IL-13Ra, GD2, NKG2D, Claudin18.2, ROR1, EGFRvIII, CS1, BCMA or GPRC5D, more preferably targeting CD19, Claudin18.2, MSLN, ROR1, GPRC5D, CD7 and BCMA. In an embodiment, the chimeric antigen receptor further comprises a signal peptide, a hinge region, or both. For the definitions of the transmembrane domain, co-stimulatory domain, primary signaling domain, and the optional hinge region, signal peptide and other structures that can be used in the CAR of the present disclosure are defined in above "immunosuppressive molecules" section.

In an embodiment, the CAR of the present disclosure may further comprise a switch structure to regulate the expression time of the CAR. For example, the switch structure may be in the form of a dimerization domain, which undergoes a conformational change by binding to its corresponding ligand, exposing the extracellular binding domain, allowing it to bind to the targeted antigen, thereby activating the signal transduction pathway. Alternatively, switch domains may also be used to link the binding domain and the signaling domain, respectively, and only when the switch domains are coupled with each other (e.g., in the presence of an inducing compound), the binding domain and the signaling domain can be linked together through the dimerization, thereby activating the signaling pathway. The switch structure may further be in the form of a masking peptide. The masking peptide can mask the extracellular binding region and prevent it from binding to the targeted antigen. When the masking peptide is cleaved by, for example, protease, the extracellular binding region can be exposed, making it a "normal" CAR structure. Various switch structures known to those skilled in the art can be used in the present disclosure.

In an embodiment, the CAR of the present disclosure may further comprise a suicide gene, i.e., to make it express a cell death signal that can be induced by an exogenous substance, so as to eliminate CAR cells when needed (e.g., when severe toxic side effects occur). For example, the suicide gene may be in the form of inserted epitopes, for example, CD20 epitopes, RQR8, etc., and when needed, CAR cells can be eliminated by adding antibodies or reagents targeting these epitopes. The suicide gene may also be herpes simplex virus thymidine kinase (HSV-TK), which causes cell death induced by ganciclovir treatment. The suicide gene may further be iCaspase-9, and the dimerization of iCaspase-9 can be induced by a chemically inducing medicament, e.g., AP1903, AP20187, etc., thereby activating the downstream Caspase3 molecule and leading to cell apoptosis. Various suicide genes known to those skilled in the art can all be used in the present disclosure.

### Engineered cells

In an embodiment, the engineered cell of the present disclosure is an engineered immune cell.

As used herein, the term "immune cell" refers to any cell of the immune system that has one or more effector functions (e.g., cytotoxic cell killing activity, secretion of cytokines, induction of ADCC and/or CDC). For example, the immune cell may be a B cell, a T cell, a macrophage, a dendritic cell, a monocyte, an NK cell, or an NKT cell. The immune cell may be obtained from a variety of sources, for example from a subject (e.g., isolated from peripheral blood monocytes, bone marrow, lymph node tissue, cord blood, thymus tissue, tissue from sites of infection, ascites, pleural effusion, spleen tissue, tumors, etc. of the subject), or from a cell line cultured in vitro (e.g., Jurkat, SupT1, NK92, etc.), or differentiated from stem cells (e.g., derived from cord blood stem cells, progenitor cells, bone marrow stem cells, hematopoietic stem cells, adult stem cells, embryonic stem cells, pluripotent stem cells, iPSCs, etc.). Preferably, the immune cell is a T cell or an NK cell, more preferably a T cell. The T cell also may be concentrated or purified. The T cell may be at any stage of development including, but not limited to, a CD4+CD8+ T cell, a CD4+ T cell (e.g., Th1 and Th2 cells), CD8+ T cell (e.g., cytotoxic T cell), a CD4-CD8- T cell, a tumor infiltrating cell, a memory T cell, a naive T cell, a γδ-T cell, an αβ-T cell, etc. In a preferred embodiment, the immune cell is a human T cell. The T cell may be isolated from the blood of a subject using a variety of techniques known to those of skill in the art, e.g., Ficoll.

In an embodiment, the engineered cell of the present disclosure is an engineered stem cell or is differentiated from the stem cell. Examples of stem cell include, but are not limited to, a cord blood stem cell, a progenitor cell, a bone marrow stem cell, a hematopoietic stem cell, an adult stem cell, an embryonic stem cell, a pluripotent stem cell, an iPSC, etc.

In an embodiment, the endogenous HLA class I genes and/or HLA class II genes of the engineered cell of the present disclosure have not been modified. In an embodiment, the endogenous HLA class I genes of the engineered cell of the present disclosure have not been modified. In an embodiment, the endogenous HLA class II genes of the engineered cell of the present disclosure have not been modified. In an embodiment, the endogenous HLA class I genes and HLA class II genes of the engineered cell of the present disclosure have not been modified. In an embodiment, the expression of at least one of the endogenous HLA class I gene and/or at least one of the endogenous HLA class II gene of the engineered cell of the present disclosure is down-regulated. In an embodiment, the expression of at least one of the endogenous HLA class I gene of the engineered cell of the present disclosure is down-regulated. In an embodiment, the expression of at least one of the endogenous HLA class II gene of the engineered cell of the present disclosure is down-regulated. In an embodiment, the expression of at least one of the endogenous HLA class I gene and at least one of the endogenous HLA class II gene of the engineered cell of the present disclosure is down-regulated. In an embodiment, the expression of at least one of the endogenous TCR/CD3 gene of the engineered cell of the present disclosure is down-regulated. In an embodiment, the expression of at least one of the endogenous TCR/CD3 gene and at least one of the endogenous HLA class I gene of the engineered cell of the present disclosure is down-regulated. In an embodiment, the expression of at least one of the endogenous TCR/CD3 gene and at least one of the endogenous HLA class II gene of the engineered cell of the present disclosure is down-regulated. In an embodiment, the expression of at least one of the endogenous TCR/CD3 gene, at least one of the endogenous HLA class I gene and at least one of the endogenous HLA class II gene of the engineered cell of the present disclosure is down-regulated. Preferably, the HLA class I gene is selected from HLA-A, HLA-B, HLA-C and B2M. Preferably, the HLA class II gene is selected from HLA-DP, HLA-DQ, HLA-DR, TAP1, TAP2, LMP2, LMP7, RFX5, RFXAP, RFXANK and CIITA, preferably selected from RFX5, RFXAP, RFXANK and CIITA. Preferably, the TCR/CD3 gene is selected from TRAC, TRBC, CD3γ, CD3δ, CD3ε and CD3ζ.

In an embodiment, the engineered cell of the present disclosure is an allogeneic cell. As used herein, the term "allogeneic" refers to any material derived from a different animal or a different patient of the same species as the individual into whom the material is introduced. Two or more individuals are considered allogeneic to each other when the genes at one or more loci differ. In some cases, allogeneic material from individuals of the same species may be genetically different enough for antigenic interactions to occur.

### Pharmaceutical composition

The present disclosure further provides a pharmaceutical composition, which comprises the engineered cell of the present disclosure as an active agent, and one or more pharmaceutically acceptable excipients. Therefore, the present disclosure further encompasses use of the engineered cell in the preparation of a pharmaceutical composition or a medicament.

As used herein, the term "pharmaceutically acceptable excipient" refers to a vector and/or excipient that is pharmacologically and/or physiologically compatible (i.e., capable of triggering a desired therapeutic effect without causing any undesired local or systemic effects) with the subject and active ingredient, and it is well known in the art (see, e.g., Remington's Pharmaceutical Sciences. Edited by Gennaro AR, 19th ed. Pennsylvania: Mack Publishing Company, 1995). Examples of pharmaceutically acceptable excipient include, but are not limited to, filler, binder, disintegrant, coating agent, adsorbent, anti-adherent, glidant, antioxidant, flavoring agent, colorant, sweetener, solvent, co-solvent, buffer agent, chelating agent, surfactant, diluent, wetting agent, preservative, emulsifier, cladding agent, isotonic agent, absorption delaying agent, stabilizer, and tension regulator. It is known to those skilled in the art to select a suitable excipient to prepare the desired pharmaceutical composition of the present disclosure. Exemplary excipients for use in the pharmaceutical composition of the present disclosure include saline, buffered saline, dextrose, and water. Generally, the selection of a suitable excipient depends, in particular, on the active agent used, the disease to be treated, and the desired dosage form of the pharmaceutical composition.

The pharmaceutical composition according to the present disclosure is suitable for multiple routes of administration. Generally, the administration is parenterally accomplished. Parenteral delivery methods include topical, intraarterial, intramuscular, subcutaneous, intramedullary, intrathecal, intraventricular, intravenous, intraperitoneal, intrauterine, intravaginal, sublingual, or intranasal administration.

The pharmaceutical composition according to the present disclosure also may be prepared in various forms, e.g., solid, liquid, gaseous or lyophilized forms, particularly the pharmaceutical composition can be prepared in the form of ointment, cream, transdermal patch, gel, powder, tablet, solution, aerosol, granule, pill, suspension, emulsion, capsule, syrup, elixir, extract, tincture or liquid extract, or in a form particularly suitable for the desired method of administration. Processes known in the present disclosure for producing a medicament may include, for example, conventional mixing, dissolving, granulating, sugar-coating, grinding, emulsifying, encapsulating, embedding or lyophilizing process. The pharmaceutical composition comprising, for example, the immune cell as described herein is generally provided in a form of solution, and preferably comprises a pharmaceutically acceptable buffer agent.

The pharmaceutical composition according to the present disclosure further may be administered in combination with one or more other agents (biological agents, e.g., antibody reagents, and/or small molecules) or treatment methods (e.g., surgery, chemotherapy or radiotherapy) suitable for the treatment and/or prevention of diseases to be treated. Preferred examples of agent suitable for the combination include known anti-cancer medicaments, e.g., cisplatin, maytansine derivatives, rachelmycin, calicheamicin, docetaxel, etoposide, gemcitabine, ifosfamide, irinotecan, melphalan, mitoxantrone, sorfimer sodiumphotofrin II, temozolomide, topotecan, trimetreate glucuronate, auristatin E, vincristine and doxorubicin; peptide cytotoxins, e.g., ricin, diphtheria toxin, pseudomonas exotoxin A, DNase and RNase; radionuclides, e.g., iodine 131, rhenium 186, indium 111, iridium 90, bismuth 210, bismuth 213, actinides 225 and astatine 213; prodrugs, e.g., antibody-directed enzyme prodrugs; immunostimulatory agents, e.g., platelet factor 4, melanoma growth stimulating protein, etc.; antibodies or fragments thereof, e.g., anti-CD3 antibodies or fragments thereof, complement activators, heterologous protein domains, homologous protein domains, viral/bacterial protein domains and viral/bacterial peptides. In addition, the pharmaceutical composition of the present disclosure also can be used in combination with one or more other treatment methods, for example, chemotherapy and radiotherapy.

In an embodiment, the pharmaceutical composition of the present disclosure is used for the treatment of a subject with cancer, infection or autoimmune diseases.

In an embodiment, the cancer is a cancer associated with the expression of a target binding to a functional exogenous receptor. For example, the cancer includes, but is not limited to, brain glioma, blastoma, sarcoma, leukemia, basal cell carcinoma, biliary tract cancer, bladder cancer, bone cancer, brain and CNS cancer, breast cancer, peritoneal cancer, cervical cancer, choriocarcinoma, colon and rectal cancer, connective tissue cancer, cancer of the digestive system, endometrial cancer, esophageal cancer, eye cancer, head and neck cancer, gastric cancer (including gastrointestinal cancer), glioblastoma (GBM), liver cancer, hepatoma, intraepithelial neoplasia, kidney cancer, laryngeal cancer, liver tumor, lung cancer (e.g., small cell lung cancer, non-small cell lung cancer, adenocarcinoma of the lung, and squamous cell lung cancer), lymphoma (including Hodgkin's lymphoma and non-Hodgkin's lymphoma), melanoma, myeloma, neuroblastoma, oral cancer (e.g., lip, tongue, mouth, and pharynx), ovarian cancer, pancreatic cancer, prostate cancer, retinoblastoma, rhabdomyosarcoma, colorectal cancer, cancer of the respiratory system, salivary gland cancer, skin cancer, squamous cell carcinoma, gastric cancer, testicular cancer, thyroid cancer, uterine or endometrial cancer, malignancy of the urinary system, vulvar cancer, and other cancers and sarcomas, and B-cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cleaved cell NHL, large mass disease NHL), mantle cell lymphoma, AIDS-related lymphoma, and Waldenstrom macroglobulinemia, chronic lymphocytic leukemia (CLL), acute lymphoblastic leukemia (ALL), B-cell acute lymphoblastic leukemia (B-ALL), T-cell acute lymphoblastic leukemia (T-ALL), B-cell prolymphocytic leukemia, blastic plasmacytoid dendritic cell neoplasm, Burkitt's lymphoma, diffuse large B-cell lymphoma, follicular lymphoma, chronic myeloid leukemia (CML), malignant lymphoproliferative disease, MALT lymphoma, hairy cell leukemia, marginal zone lymphoma, multiple myeloma, myelodysplasia, plasmablastic lymphoma, preleukemia, plasmacytoid dendritic cell neoplasm, and post-transplant lymphoproliferative disorder (PTLD); and other diseases associated with target expression. Preferably, the disease that can be treated with the engineered immune cell or the pharmaceutical composition of the present disclosure is selected from the group consisting of: leukemia, lymphoma, multiple myeloma, brain glioma, pancreatic cancer, gastric cancer, etc.

In an embodiment, the infection includes, but is not limited to, infections caused by viruses, bacteria, fungi, and parasites.

In an embodiment, the autoimmune disease includes, but is not limited to type I diabetes, celiac disease, Graves' disease, inflammatory bowel disease, multiple sclerosis, psoriasis, rheumatoid arthritis, Addison's disease, Sjögren's syndrome, Hashimoto's thyroiditis, myasthenia gravis, vasculitis, pernicious anemia and systemic lupus erythematosus, etc.

The present disclosure will be described in detail below with reference to the accompanying drawings and examples. It should be noted that those skilled in the art should understand that the drawings of the present disclosure and the examples thereof are only for the purpose of illustration, and shall not constitute any limitation to the present disclosure. In the case of no contradiction, the examples in the present application and the features in the examples can be combined with each other.

### BRIEF DESCRIPTION OF DRAWINGS

Figure 1: shows the killing activity of Fas-knockout CAR-T cells against target cells.
Figure 2: shows the cytokine release levels of Fas-knockout CAR-T cells (A: CD19-targeting CAR-T cells; B: Claudin18.2-targeting CAR-T cells) after co-incubation with target cells.
Figure 3: shows the suppressive effect of Fas-knockout CAR-T cells (A: CD19-targeting CAR-T cells; B: Claudin18.2-targeting CAR-T cells) against T cell killing.
Figure 4: shows the in vivo tumor suppressive effect of Fas-knockout CAR-T cells.
Figure 5: shows the killing activity of TGFBR1- or TGFBR2-knockout CAR-T cells (A: CAR-T cells comprising immunosuppressive molecules targeting NKG2A and PD1 B: CAR-T cells comprising FasL-targeting immunosuppressive molecules) against target cells.
Figure 6: shows the cytokine release levels of TGFBR1- or TGFBR2-knockout CAR-T cells after co-incubation with target cells (A: IL2 and IFN-γ release levels of CAR-T cells comprising immunosuppressive molecules targeting NKG2A and PD1; B: IL2 release levels of CAR-T cells comprising FasL-targeting immunosuppressive molecules; C: IFN-γ release levels of CAR-T cells comprising FasL-targeting immunosuppressive molecules).
Figure 7: shows the in vivo tumor suppressive effect of TGFBR1- or TGFBR2-knockout CAR-T cells (A: CAR-T cells comprising immunosuppressive molecules targeting NKG2A and PD1; B: CAR-T cells comprising FasL-targeting immunosuppressive molecules).

### DETAILED DESCRIPTION

### Structures of exemplary CARs and immunosuppressive molecules used in the following examples:

The CAR structure comprises an extracellular domain, a CD8α hinge region (SEQ ID NO: 27), a CD8α transmembrane region (SEQ ID NO: 15), a 4-1BB co-stimulatory domain (SEQ ID NO: 18), and a CD3ζ intracellular region (SEQ ID NO: 21) linked in sequence. The extracellular domain comprised in the CD19 CAR structure is anti-CD19 scFv (SEQ ID NO: 13). The extracellular domain comprised in the Claudin18.2 CAR structure is anti-Claudin18.2 vhh (SEQ ID NO: 4).

The structure of the PD1-targeting immunosuppressive molecule IIM-PDL1 comprises a PDL1 signal peptide (SEQ ID NO: 53), an extracellular region of PDL1 (SEQ ID NO: 55), a PDL1 transmembrane region (SEQ ID NO: 54), and a CD28 co-stimulatory domain (SEQ ID NO: 17) linked in sequence.

The structure of the NKG2A-targeting immunosuppressive molecule IIM-NKG2A comprises anti-NKG2A scFv (SEQ ID NO: 41), an IgG4 hinge region (SEQ ID NO: 29), a CD28 transmembrane region (SEQ ID NO: 14), and a CD28 co-stimulatory domain (SEQ ID NO: 17) linked in sequence.

The structure of the FasL-targeting immunosuppressive molecule IIM-FasL comprises a B2M signal peptide (SEQ ID NO: 23), an anti-FasL scFv (SEQ ID NO: 67), an IgG4 hinge region (SEQ ID NO: 29), a CD28 transmembrane region (SEQ ID NO: 14), and a CD28 co-stimulatory domain (SEQ ID NO: 17) linked in sequence.

### Example 1. Construction of CAR-T immune cells of the present disclosure

The coding sequence of CD19 CAR was synthesized and cloned into a pGEM-T Easy vector (Promega), yielding a CAR19 plasmid. The CAR19 plasmid further comprised the coding sequence of the PD1-targeting immunosuppressive molecule IIM-PDL1 linked via T2A (SEQ ID NO: 30), yielding the CAR19P plasmid,

The coding sequence of Claudin18.2 CAR was synthesized and cloned into a pGEM-T Easy vector (Promega), yielding a CAR18.2 plasmid. The CAR18.2 plasmid further comprised the coding sequence of the PD1-targeting immunosuppressive molecule IIM-PDL1 and the coding sequence of the NKG2A-targeting immunosuppressive molecule IIM-NKG2A, each linked via T2A(SEQ ID NO: 30), yielding the CAR18.2AP plasmid, The CAR18.2 plasmid further comprised the coding sequence of the FasL-targeting immunosuppressive molecule IIM-FasL linked via T2A (SEQ ID NO: 30), yielding the CAR18.2FL plasmid,

Three ml Opti-MEM (Gibco) was added to a sterile tube to dilute the above plasmid, and then packaging vector psPAX2 (Addgene) and envelope vector pMD2.G (Addgene) were added according to the ratio of plasmid : viral packaging vector : viral envelope vector = 4:2:1. Then, 120 µl X-treme GENE HP DNA transfection reagent (Roche) was added, mixed immediately, and incubated at room temperature for 15 min, and then the plasmid/vector/transfection reagent mixture was added dropwise to the culture flask of 293T cells. Viruses were collected at 24 hours and 48 hours, pooled, and ultracentrifuged (25000 g, 4°C, 2.5 hours) to obtain concentrated lentiviruses.

T cells were activated with DynaBeads CD3/CD28 CTSTM (Gibco), and were further cultured for 1 day under 37°C and 5% CO₂. On the second day, the concentrated lentiviruses were added, and after 3 days of continuous culture, T cells expressing CD19 CAR (CAR19), T cells expressing CD19 CAR and IIM-PDL1 combination (CAR19P), T cells expressing Claudin18.2 CAR and IIM-FasL (CAR18.2FL), and T cells expressing Claudin18.2 CAR and two immunosuppressive molecules (IIM-PDL1+IIM-NKG2A) combination (CAR18.2AP) were obtained. Additionally, the above lentiviruses was used to transfect T cells in which Fas gene had been knocked out by the CRISPR/Cas9 system (CAR19, CAR19P, and CAR18.2AP), yielding CAR19-Fas KO T cells, CAR19P-Fas KO T cells, and CAR18.2AP-Fas KO T cells.

### Example 2. Killing effects of CAR-T cells on target cells

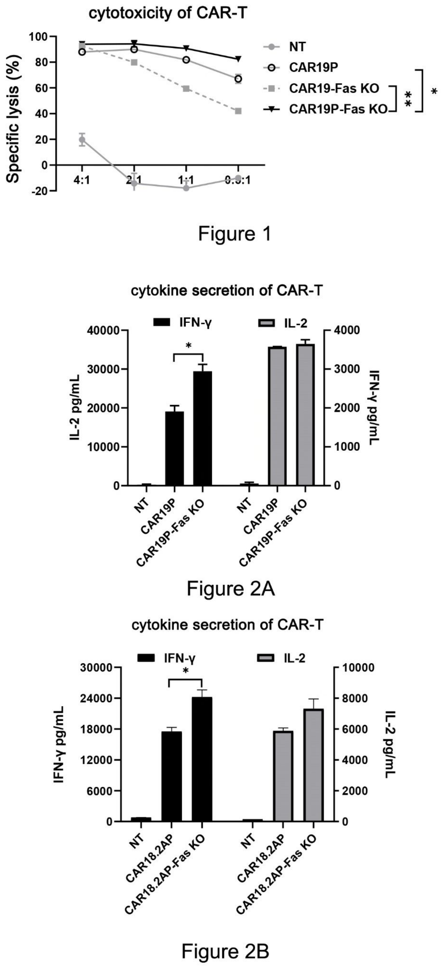

Target cells, Raji cells, were plated into 96-well plates at a concentration of 1×10⁴ cells/well, and then, NT cells, CAR19P cells, CAR19-Fas KO T cells and- CAR19P-Fas KO T cells were plated into 96-well plates at an effector-to-target ratio of 4:1, 2:1, 1:1 or 0.5:1 for co-culture. After 18 hours, the fluorescence value was measured with a microplate reader. According to the calculation formula: (average fluorescence value of target cells - average fluorescence value of samples)/average fluorescence value of target cells × 100%, the killing efficiency was calculated, and the results are shown in Figure 1.

It can be seen that CAR19P T cells, CAR19-Fas KO T cells and CAR19P-Fas KO T cells all showed effective specific killing on target cells, and compared with the first two, the killing activity of CAR19P-Fas KO T cells was significantly improved. This indicates that the combination of down-regulating Fas genes and expressing immunosuppressive molecules produces a synergistic effect, which can maximize the killing effect of CAR19 T cells.

### Example 3. Cytokine release levels of CAR-T cells after co-incubation with target cells

Target cells (Raji cells) were plated in 96-well plates at a concentration of 1×10⁵ cells/well, NT cells, CAR19P T cells, and CAR19P-Fas KO T cells were added at a ratio of 1:1, respectively, and the cell co-culture supernatant was collected after 18-24 hours of co-culture. Human IL-2 DuoSet ELISA Kit (R&D systems) and Human IFN-gamma DuoSet ELISA Kit (R&D systems) were used to detect the contents of IL2 and IFN-γ in the co-culture supernatant, respectively, and the results are shown in Figure 2A. It can be seen that, compared with NT cells, the two CAR-T cells showed significantly increased release levels of cytokines IL-2 and IFN-γ after co-culture with target cells, indicating that this cytokine release is specific. In addition, knockout of Fas genes significantly increased the IFNγ release level of CAR19 T cells.

The same method was used to detect the cytokine release levels of Claudin18.2-targeting CAR-T cells targeting after co-incubation with target cells NUGC4-18.2, and the results are shown in Figure 2B. Similarly, knockout of Fas genes significantly increased the IFNγ release level of CAR18.2 T cells.

### Example 4. Knockout of Fas genes significantly enhances CAR-T cell survival

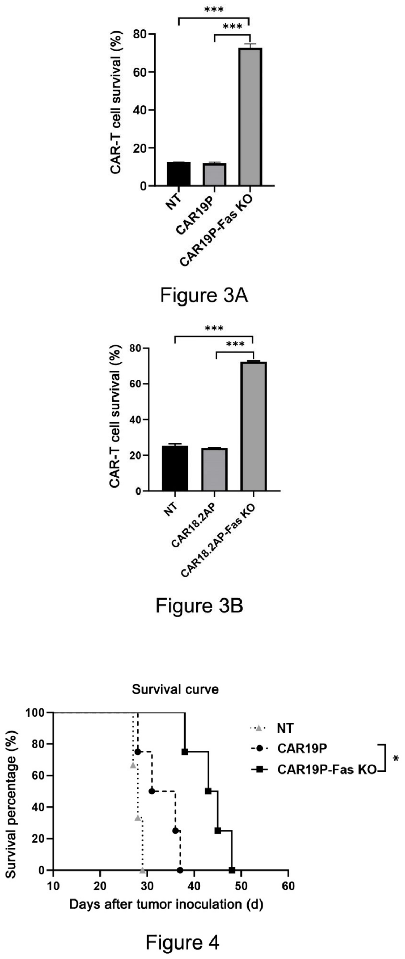

In order to verify the ability of CAR-T cells with down-regulated Fas gene expression therein to resist killing, the CAR-T cells of the present disclosure were first plated into 96-well plates at a concentration of 1×10⁵ cells/well, then effector cell CAR7 T cells (T cells expressing CD7 CAR, labeled with Far-red) were also plated into 96-well plates at an effector-to-target ratio of 1:1 for co-culture, and after 24 hours, the survival ratio of the CAR-T cells of the present disclosure was detected by flow cytometry. The results are shown in Figure 3A ( CD19-targeting) and Figure 3B ( Claudin18.2-targeting).

It can be seen that the survival ratios of CAR19P-T cells and CAR18.2AP-T cells that only express immunosuppressive molecules are comparable to those of NT cells, and further knockout of Fas genes can significantly improve the survival of CAR-T cells.

### Example 5. In vivo suppressive effect of CAR T cells on tumors

Fifteen healthy female NPI mice aged 6-8 weeks were divided into 3 groups, with 5 mice in each group: NT cells, CAR19P-T cells, and CAR19P-Fas KO T cells. On day 0, 5×10⁵ Raji cells were injected into the tail vein of each mouse. Six days later, 2x10⁶ NT cells or corresponding CAR-T cells were injected into the tail vein of each mouse according to the grouping. The status of the mice was assessed weekly. The mouse survival curve is shown in Figure 4.

It can be seen that the survival rate of mice treated with CAR19P-T cells was significantly higher than that of the control group, and the CAR19P-Fas KO T cell group with Fas gene knockout showed the longest survival period. This indicates that knockout of endogenous Fas genes can enhance the in vivo killing activity of CAR-T cells.

### Example 6. Construction of CAR-T cells with TGFBR1- or TGFBR2-knockout and expressing the immunosuppressive molecules and verification of the function thereof

CAR-T cells with TGFBR1- or TGFBR2-knockout therein and expressing immunosuppressive molecules were constructed according to the method of Example 1. Specifically, after the CAR18.2AP and CAR18.2FL plasmids prepared in Example 1 were packaged into lentiviruses, they were used to transfect T cells in which TGFBR1 had been knocked out by the CRISPR/Cas9 system, yielding CAR18.2AP-TR1 KO T cells and CAR18.2FL-TR1 KO T cells; or to transfect T cells in which TGFBR2 had been knocked out by the CRISPR/Cas9 system were transfected, yielding CAR18.2AP-TR2 KO T cells and CAR18.2FL-TR2 KO T cells.

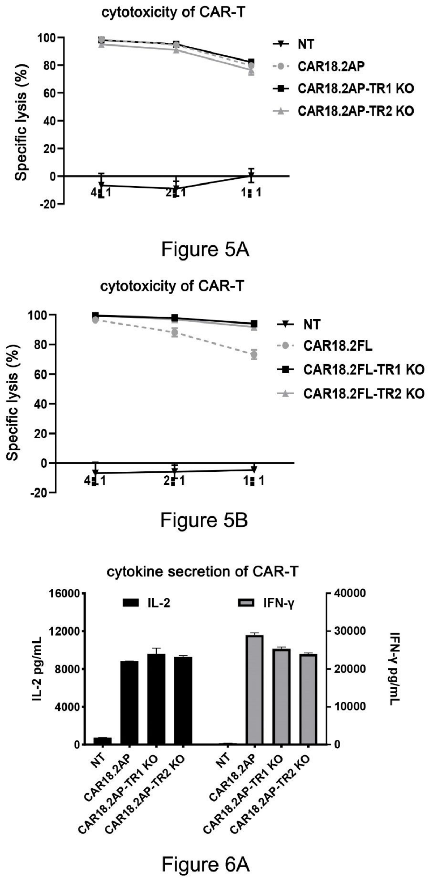

The killing activity of the above CAR-T cells was detected by the method of Example 2, and the results are shown in Figure 5A (CAR-T cells comprising immunosuppressive molecules targeting NKG2A and PD1) and Figure 5B (CAR-T cells comprising FasL-targeting immunosuppressive molecules). It can be seen that compared with CAR18.2AP T cells, additional knockout of TGFBR1 or TGFBR2 genes did not significantly affect the killing activity of CAR-T cells; compared with CAR18.2FL T cells, additional knockout of TGFBR1 or TGFBR2 genes enhanced the killing activity of CAR-T cells.

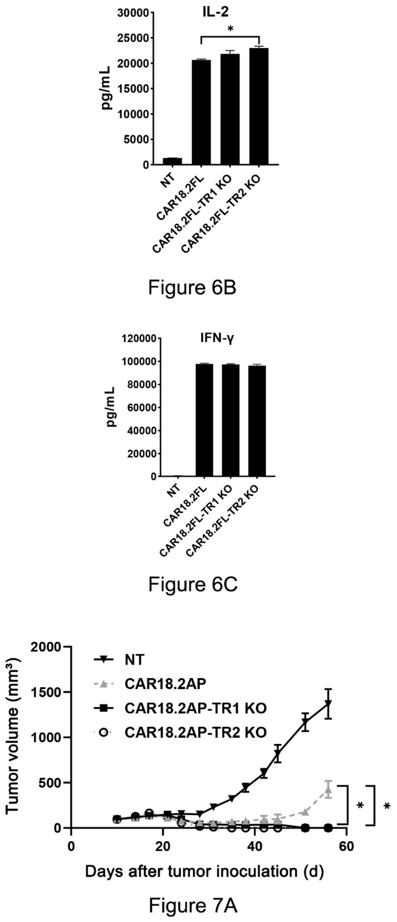

The cytokine release levels of the above CAR-T cells after co-incubation with target cells were detected by the method of Example 3, and the results are shown in Figure 6A (IL2 and IFN-γ release levels of CAR-T cells comprising immunosuppressive molecules targeting NKG2A and PD1), Figure 6B (IL2 release levels of CAR-T cells comprising FasL-targeting immunosuppressive molecules), and Figure 6C (IFN-γ release levels of CAR-T cells comprising FasL-targeting immunosuppressive molecules). It can be seen that compared with CAR18.2AP T cells or CAR18.2FL T cells, additional knockout of TGFBR1 or TGFBR2 genes did not significantly affect the release levels of cytokines, either.

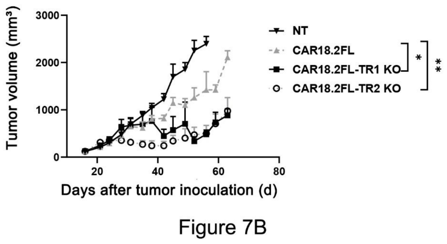

Twenty healthy female NPI mice aged 6-8 weeks were divided into 4 groups, with 5 mice in each group: NT cells, CsAR18.2AP T cells, CAR18.2AP-TR1 KO T cells, and CAR18.2AP-TR2 KO T cells. Another twenty healthy female NPI mice aged 6-8 weeks were divided into 4 groups, with 5 mice in each group: NT cells, CAR18.2FL T cells, CAR18.2FL-TR1 KO T cells, and CAR18.2FL-TR2 KO T cells. On day 0, 4×10⁶ NUGC4-18.2 cells were injected into the tail vein of each mouse. Fourteen days later, 2x10⁶ NT cells or corresponding CAR-T cells were injected into the tail vein of each mouse according to the grouping. The status of the mice was assessed weekly. The progression of tumor burden in mice is shown in Figure 7A (CAR-T cells comprising immunosuppressive molecules targeting NKG2A and PD1) and Figure 7B (CAR-T cells comprising FasL-targeting immunosuppressive molecules). It can be seen that compared with CAR18.2AP T cells, additional knockout of TGFBR1 or TGFBR2 genes significantly reduced the tumor burden in mice and maintained it at a low level until experimental termination, when almost no tumor cells were detected; compared with CAR18.2FL T cells, additional knockout of TGFBR1 or TGFBR2 genes significantly reduced the tumor burden in mice, and the effect of CAR-T cells in reducing tumor burden was more significant after knockout of TGFBR2 genes.

In summary, knocking out of endogenous Fas, TGFBR1, TGFBR2 and other genes combined with expressing immunosuppressive molecules, will on the one hand, not adversely compromise the killing activity or cytokine release characteristics of CAR-T cells (capable of producing a synergistic effect even in the case of Fas knockout combined with expression of immunosuppressive molecules, significantly improving its killing activity and IFNγ release levels), and on the other hand, compared with expressing immunosuppressive molecules alone, is capable of further significantly reducing the risk of immune rejection of CAR-T cells, improving their survival rate, and thus enhancing the efficacy of CAR-T cells in tumor suppression.

It should be noted that the above-mentioned are merely for preferred examples of the present disclosure and not used to limit the present disclosure. For those skilled in the art, various modifications and changes may be made to the present disclosure. Those skilled in the art should understand that any amendments, equivalent replacements, improvements, and so on, made within the spirit and principle of the present disclosure, should be covered within the scope of protection of the present disclosure.

## Claims

1. An engineered cell comprising:
(i) the expression of at least one of the endogenous gene selected from the group consisting of: Fas, TNFR1, DR3, DR4, DR5, TGFBR1, TGFBR2 is down-regulated; and
(ii) an exogenous immunosuppressive molecule is expressed, wherein the immunosuppressive molecule comprises one or more immunosuppressive protein-binding domains, a transmembrane domain and a co-stimulatory domain, and does not comprise a primary signaling domain, wherein the immunosuppressive protein-binding domain binds to an immunosuppressive protein selected from the group consisting of: PD1, NKG2A, FasL and CTLA4.

2. The engineered cell of claim 1, **characterized in that** the immunosuppressive protein-binding domain comprises an anti-PD1 antibody, an anti-NKG2A antibody, an anti-FasL antibody, an anti-CTLA4 antibody, an extracellular region of PDL1, an extracellular region of PDL2, an extracellular region of HLA-E, an extracellular region of Fas, an extracellular region of CD80, an extracellular region of CD86, or a combination thereof.

3. The engineered cell of claim 1 or 2, **characterized in that** the transmembrane domain is selected from a transmembrane domain of a protein selected from the group consisting of: a TCR α chain, a TCR β chain, a TCR γ chain, a TCR δ chain, a CD3 ζ subunit, a CD3 ε subunit, a CD3 γ subunit, a CD3 δ subunit, CD28, CD45, CD4, CD5, CD8a, CD9, CD16, CD22, CD33, CD37, CD47, CD64, CD80, CD86, CD94, CD134, CD137, CD154, KIRDS2, OX40, CD2, CD27, CD18, ICOS, 4-1BB, GITR, CD40, BAFFR, HVEM, SLAMF7, NKp80, CD160, BCMA, IL-2R β, IL-2R γ, IL-7R a, ITGA1, VLA1, CD49a, ITGA4, IA4, CD49D, ITGA6, VLA-6, CD49f, ITGAD, CD1 1d, ITGAE, CD103, ITGAL, CD1 la, LFA-1, ITGAM, CD1 lb, ITGAX, CD1 1c, ITGB1, CD29, ITGB2, CD18, LFA-1, ITGB7, TNFR2, DNAM1, SLAMF4, CD84, CD96, CEACAM1, CRT AM, Ly9, CD160, PSGL1, CDIOO, SLAMF6, SLAMF1, SLAMF8, CD162, LTBR, PAG/Cbp, NKp44, NKp30, NKp46, NKG2D or NKG2C.

4. The engineered cell of any one of claims 1-3, **characterized in that** the co-stimulatory domain is selected from a intracellular region of a protein selected from the group consisting of: LTB, CD94, TLR1, TLR2, TLR3, TLR4, TLR5, TLR6, TLR7, TLR8, TLR9, TLR10, CARD11, CD2, CD7, CD8, CD18, CD27, CD28, CD30, CD40, CD54, CD83, CD134, 4-1BB, CD270, CD272, B7-H3, ICOS, CD357, DAP10, DAP12, LAT, NKG2C, SLP76, PD-1, LIGHT, TRIM, ZAP70 and a combination thereof.

5. The engineered cell of any one of claims 1-4, **characterized in that** the immunosuppressive molecule does not comprise a CD3ζ intracellular region.

6. The engineered cell of claim 1, **characterized in that** the engineered cell expresses two exogenous immunosuppressive molecules.

7. The engineered cell of claim 6, **characterized in that** the two exogenous immunosuppressive molecules comprise binding domains that bind different immunosuppressive proteins.

8. The engineered cell of any one of claims 1-7, further expressing a functional exogenous receptor.

9. The engineered cell of claim 8, **characterized in that** the functional exogenous receptor is selected from the group consisting of: a chimeric antigen receptor, a chimeric T cell receptor, a T cell antigen coupler, and a T cell fusion protein, preferably a chimeric antigen receptor.

10. The engineered cell of claim 9, **characterized in that** the functional exogenous receptor comprises an extracellular domain that specifically recognizes an antigen.

11. The engineered cell of claim 10, **characterized in that** the extracellular domain comprises an antibody that specifically recognizes the antigen or a ligand of the antigen.

12. The engineered cell of claim 10, **characterized in that** the antigen is selected from the group consisting of: ALK, ADRB3, AKAP-4, APRIL, ASGPR1, BCMA, B7H3, B7H4, B7H6, ber-abl, BORIS, BST2, BAFF-R, BTLA, CD2, CD3, CD4, CD5, CD7, CD8, CD19, CD20, CD22, CD24, CD25, CD28, CD30, CD33, CD38, CD40, CD44, CD44v6, CD44v7/8, CD47, CD52, CD56, CD57, CD58, CD70, CD72, CD79a, CD79b, CD80, CD81, CD86, CD97, CD123, CD133, CD137, CD 138, CD151, CD171, CD179a, CD300LF, CLEC12A, CDH16, CSPG4, CS1, CLL-1, Claudin 6, Claudin18.1, Claudin 18.2, CEA, CEACAM6, c-Met, CAIX, CXORF61, CA125, CYP1B1, CS1, ELF2M, EGFR, EPCAM, EGFRvIII, EphA2, ERG/TMPRSS2ETS fusion gene, ETV6-AML, EMR2, EGP2, EGP40, FAP, FAR, FBP, FLT3, FOSL1, FCRL5, FCAR, Flt3, Flt4, Frizzled, GD2, GD3, gp100, gp130, GM3, GPC2, GPC3, GPRC5D, GPR20, GloboH, GHRHR, GHR, GITR, Her2, HER3, HER-4, HMWMAA, HAVCR1, HPV E6,E7, HVEM, HIV-1Gag, HLA-A1, HLA-A2, IL6R, IL-11Ra, IL-13Ra, IGF-I receptor, LTPR, LIFRP, LRP5, IGLL1, IGF1R, KIT, Kappa Light Chain, KDR, LewisY, LMP2, LY6K, LAGE-1a, legumain, LCK, LAIR1, LILRA2, LY75, MSLN, MUC1, MUC16, MAGE-A1, MAGE3, MAD-CT-1, MelanA/MART1, ML-IAP, MYCN, mut hsp70-2, NCAM, NY-BR-1, NY-ESO-1, NA17, Notch-1-4, nAchR, NKG2D, NKG2D ligand, OY-TES1, OR51E2, OX40, PRSS21, PSCA, PD1, PD-L1, PD-L2, PSMA, Prostase, PAP, PDGFR-β, PCTA-1/galectin 8, p53, p53 mutant, prostein, PLAC1, PANX3, PAX3, PAX5, PTCH1, RANK, RAGE-1, ROR1, Ras mutant, RhoC, RU1, RU2, Robol, SSEA-4, SSX2, SART3, Sp17, TSHR, Tn Ag, TGS5, TEM1/CD248, TEM7R, TARP, TCRα, TCRβ, TGFBR1, TGFBR2, TNFRSF4, TWEAK-R, TLR7, TLR9, TAG72, TROP-2, Tie 2, TRP-2, TNFR1, TNFR2, TEM1, UPK2VEGFR, WT1, XAGE1, 5T4, 8H9, αvβ6 integrin, CA9, folate receptor α, ephrin B2, tyrosinase, fucosyl GM1, o-acetyl-GD2, folate receptor β, polysialic acid, sperm protein 17, survivin and telomerase, sarcoma translocation breakpoint, human telomerase reverse transcriptase/hTERT, androgen receptor, intestinal carboxylesterase, cyclin B1, fibronectin, tenascin, carcinoembryonic variant of tumor necrosis region, or any combination thereof.

13. The engineered cell of claim 8, **characterized in that** the functional exogenous receptor is a chimeric antigen receptor targeting CD19, CD7, CD19, CD20, CD22, CD30, CD33, CD38, CD123, CD138, CD171, MUC1, MSLN, AFP, folate receptor α, CEA, PSCA, PSMA, Her2, EGFR, IL-13Ra, GD2, NKG2D, Claudin18.2, ROR1, EGFRvIII, CS1, BCMA or GPRC5D.

14. The engineered cell of any one of claims 1-13, **characterized in that** the expression of at least one of the endogenous TCR/CD3 gene of the engineered cell is suppressed or silenced.

15. The engineered cell of claim 14, **characterized in that** the TCR/CD3 gene is selected from the group consisting of: TRAC, TRBC, CD3γ, CD3δ, CD3ε, CD3ζ, and a combination thereof.

16. The engineered cell of claim 1, **characterized in that** the HLA class I genes and/or HLA class II genes of the engineered cell have not been modified.

17. The engineered cell of claim 1, **characterized in that** the expression of at least one of the endogenous HLA class I gene and/or HLA class II gene of the engineered cell is down-regulated.

18. The engineered cell of any one of claims 1-17, **characterized in that** the engineered cell is a B cell, a T cell, a macrophage, a dendritic cell, a monocyte, an NK cell, or an NKT cell.

19. The engineered immune cell of claim 18, **characterized in that** the engineered cell is a CD4+CD8+ T cell, a CD4+T cell, a CD8+T cell, a CD4-CD8-T cell, a tumor infiltrating cell, a memory T cell, a naive T cell, a γδ-T cell or an αβ-T cell.

20. The engineered cell of any one of claims 1-19, **characterized in that** the engineered cell is derived from a cord blood stem cell, a progenitor cell, a bone marrow stem cell, a hematopoietic stem cell, an adult stem cell, an embryonic stem cell, a pluripotent stem cell, and an iPSC.

21. A pharmaceutical composition comprising the engineered immune cell of any one of claims 1-20, and one or more pharmaceutically acceptable excipients.

22. The pharmaceutical composition of claim 21, **characterized in that** the pharmaceutical composition is used for the treatment of cancer, infection or autoimmune diseases.
